# EUROPEAN PATENT APPLICATION

(11) **EP 4 484 574 A1**
(43) Date of publication of application: **01.01.2025**
(21) Application number: 22927705.8
(22) Date of filing: 24.02.2022
(51) Int. Cl.: C12Q 1/6869, C12Q 1/6806, C40B 50/06

(54) **SINGLE-CELL TRANSCRIPTOME SEQUENCING METHOD AND USE THEREOF**

(71) Applicant: Zhejiang University, Hangzhou, Zhejiang 310058 (CN)
(72) Inventor: WANG, Yongcheng, Hangzhou, Zhejiang 311121 (CN); XU, Ziye, Hangzhou, Zhejiang 311121 (CN); WANG, Yuting, Hangzhou, Zhejiang 311121 (CN)
(74) Representative: Brann AB
(86) International application number: PCT/CN2022/077609
(87) International publication number: WO 2023/159416

(57) **Abstract**

The present invention provides a single-cell transcriptome sequencing method and use thereof. The method comprises: preparing a single-cell suspension with a test cell sample, then fixing cells with a fixative solution; and using a reverse transcription primer to perform an in-situ reverse transcription reaction on RNA of the fixed single cell to synthesize a first cDNA strand.

## Description

### TECHNICAL FIELD

The present disclosure pertains to the field of biotechnologies and relates to the field of single-cell sequencing, and specifically, to a single-cell transcriptome sequencing method and use thereof.

### BACKGROUND

At present, the most widely used high-throughput single-cell transcriptome sequencing technique is a single-cell sequencing technique that is developed by 10X Genomics and that is based on a droplet microfluidic platform. Through this technique, thousands of cells can be labeled, sequenced, and analyzed, and gene expression profiles on a single-cell level can be obtained, thereby dividing cell subsets, and detecting differential expression genes among the cell subsets. Similar techniques include inDrop technique and Drop-seq technique.

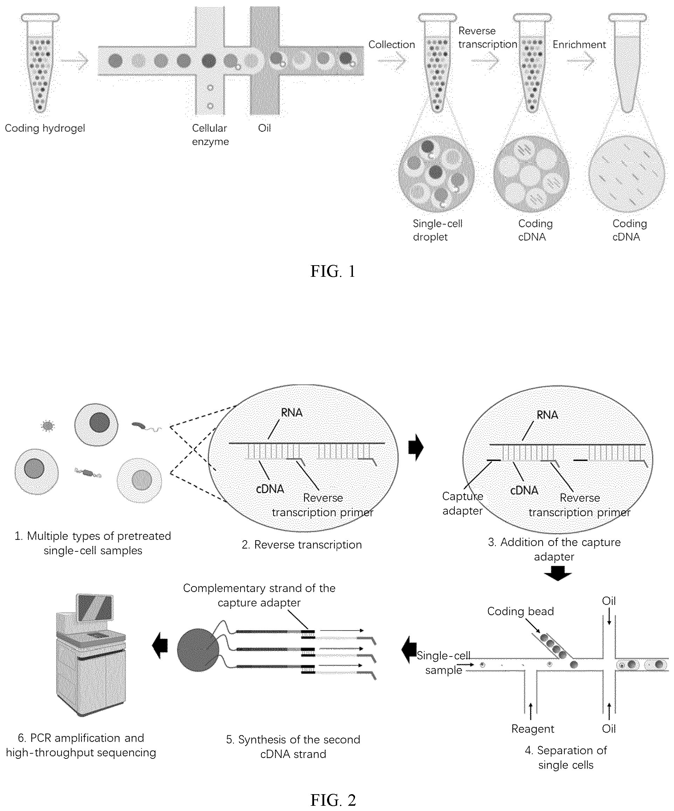

A basic operation and principle of the transcriptome sequencing technique developed by 10X Genomics (referring to FIG. 1) are as follows: A sample is prepared into a single cell suspension (with cell viability to be higher than 90% and a cell concentration of generally about 700 cells/pL to 1200 cells/µL), and then a single coding bead (the coding bead is formed by a gel bead and a piece of primer attached to the gel bead) with a barcode and a primer is wrapped in an oil droplet together with a single cell through the microfluidic platform; in each single droplet, the gel bead is dissolved, the cell is lysed to release mRNA, and cDNA that is used for sequencing and that has the barcode and UMI information is generated by reverse transcription; and after a droplet oil layer is disrupted, cDNA is collected for amplification, cDNA library is prepared, and then the library is sequenced and detected by using Illumina sequencing platform, to obtain a great amount of gene expression data of single cells.

The primer sequence attached to the gel bead used in the foregoing sequencing technique includes four parts: Illumina TruSeq Read 1 sequencing primer, a 16-nt barcode, a 12-nt UMI, and 30-nt Poly(dT) reverse transcription primer. The TruSeq Read 1 sequencing primer is a known short peptide nucleotide sequence for subsequent sequencing via instruments. The barcodes are in a one-to-one correspondence with beads and there are a total of 4 million barcodes. The UMI is a sequence formed by random bases, and each DNA molecule has its own UMI sequence for distinguishing between different samples (that is, determining which sequences obtained via sequencing are derived from the same original cDNA molecule) during mixed sequencing. The Poly(dT) reverse transcription primer is a homopolymeric DNA fragment including 30 thymine bases, and is used to capture transcripts with poly(A) tails.

In the representative high-throughput single-cell transcriptome sequencing technique developed by 10X Genomics, the used reverse transcription primers are all Poly(dT) reverse transcription primers, and therefore, only a part of 3' end transcript information can be obtained, and RNA without poly(A) (including damaged mRNA fragments and miRNA, IncRNA, and the like without poly(A)) cannot be detected. As a result, the technique is of very low sensitivity in actual application, and usually, only less than 10% of mRNA is detectable. In addition, in this technique, there are high quality requirements for RNA. For better sequencing results, cell viability of a to-be-sequenced sample needs to be greater than 80%. A sequencing effect is usually very poor when a cryopreserved or fixed sample is used. In addition, the existing high-throughput single-cell transcriptome sequencing technique cannot be used for sequencing transcriptomes of bacteria because RNA of bacteria includes no poly(A).

### SUMMARY

In order to solve the foregoing problem, according to the first aspect, an objective of the present disclosure is to provide a single-cell transcriptome sequencing method, comprising: preparing a single-cell suspension with a test cell sample and then fixing cells with a fixative solution; and synthesizing a first cDNA strand using by performing an in-situ reverse transcription reaction on RNA of the fixed single cell using a reverse transcription primer.

In a specific embodiment, the reverse transcription primer in the present disclosure may be a random reverse transcription primer, a reverse transcription primer designed for a target RNA sequence, or a mixture of the random reverse transcription primer and the reverse transcription primer designed for the target RNA sequence. Preferably, the reverse transcription primer is the random reverse transcription primer.

In a specific embodiment, the method in the present disclosure may further comprise adding a capture adapter to an end of the first cDNA strand obtained by the reverse transcription, where the capture adapter is sequence-complementary to a complementary strand of the capture adapter on the coding bead; and the capture adapter is a random fragment of a known sequence, and preferably, the capture adapter is Poly(dA) fragment, Poly(dT) fragment, Poly(dG) fragment, or Poly(dC) fragment.

In a specific embodiment, the method in the present disclosure may further comprise embedding the single cell and the single coding bead into a single chamber after adding the capture adapter to separate the single cell and synthesize a second cDNA strand in the single chamber.

In a specific embodiment, the method in the present disclosure may further comprise amplifying double-stranded cDNA via PCR, and constructing a library and performing sequencing, after the second cDNA strand is synthesized.

In a specific embodiment, single-stranded DNA on the coding bead used in the method in the present disclosure may comprise a complementary fragment of an upstream amplification primer, a barcode, a UMI, and a complementary strand of the capture adapter; and preferably, the barcode may include one or more barcodes, or preferably, three barcodes.

In a specific embodiment, a microfluidic chip or a microwell may be used in the method in the present disclosure to complete the separation of the single cells.

In a specific embodiment, the fixative solution used in the method in the present disclosure may be a simple fixative solution or a mixed fixative solution; preferably, the simple fixative solution may comprise, but not limited to, paraformaldehyde, formaldehyde, formalin, methanol, acetone, ethanol, acetic acid, picric acid, chromic acid, potassium dichromate and mercury bichloride; and preferably, the mixed fixative solution may comprise, but not limited to, acetic acid-alcohol mixture, formalin-acetic acid-alcohol solution, and Bouin's fixative solution.

In the specific embodiments, the method in the present disclosure comprises: preparing the single-cell suspension with the test cell sample and then fixing cells with the fixative solution; synthesizing the first cDNA strand using by performing the in-situ reverse transcription reaction on RNA of the fixed single cell using the reverse transcription primer; adding the capture adapter to the end of the first cDNA strand obtained by the reverse transcription, where the capture adapter is sequence-complementary to the complementary strand of the capture adapter on the coding bead; embedding the single cell and the single coding bead into the single chamber after adding the capture adapter to separate the single cells; synthesizing the second cDNA strand in the single chamber to form the double-stranded DNA; and amplifying the obtained double-stranded cDNA via the PCR, constructing the library and performing sequencing.

According to the second aspect, an objective of the present disclosure is to provide use of the method in the first aspect in whole-transcriptome sequencing of a single cell, a single nucleus, and a single microorganism; and preferably, the use may be the use in fields of microbiology, basic medicine, clinical medicine, agronomy, cell biology, immunology, developmental biology, pathology, neurobiology and neurodevelopment, genetics, stem cells, tumors, reproductive health, metagenomics, microecology, and new drug development.

According to a third aspect, an objective of the present disclosure is to provide a diagnostic or therapeutic method comprising: performing gene sequencing in the single-cell transcriptome sequencing method in the first aspect.

Compared with the prior art, the sequencing method in the present disclosure has the following advantages:
(1) A greater variety of cell samples can be tested in the method in the present disclosure, and the method can be applied to single cells and single nuclei of eukaryotic cells, prokaryotes (bacteria, actinomycetes, rickettsiae, chlamydiae, mycoplasma, cyanobacteria, archaebacteria, and the like), single-celled algae, viruses, and the like.
(2) The method in the present disclosure can be applied to a fixed sample, a cryopreserved sample, a paraffin-embedded sample, and the like with low cell viability, in addition to a fresh sample.
(3) In the method in the present disclosure, coding RNA and various forms of non-coding RNA can be detected, a complete transcriptome map can be obtained, and analyses of quantification on a transcriptome level, gene differential expression, alternate splicing, gene fusion, RNA interaction, and the like can be performed.
(5) Compared with the existing microfluidic single-cell RNA sequencing technique, RNA detection sensitivity in the method in the present disclosure is higher under the same sequencing depth.

### BRIEF DESCRIPTION OF DRAWINGS

FIG. 1 is a schematic diagram of a principle of an existing single-cell transcriptome sequencing method developed by 10X Genomics.
FIG. 2 is a schematic diagram of a principle of a single-cell transcriptome sequencing method in the present disclosure.

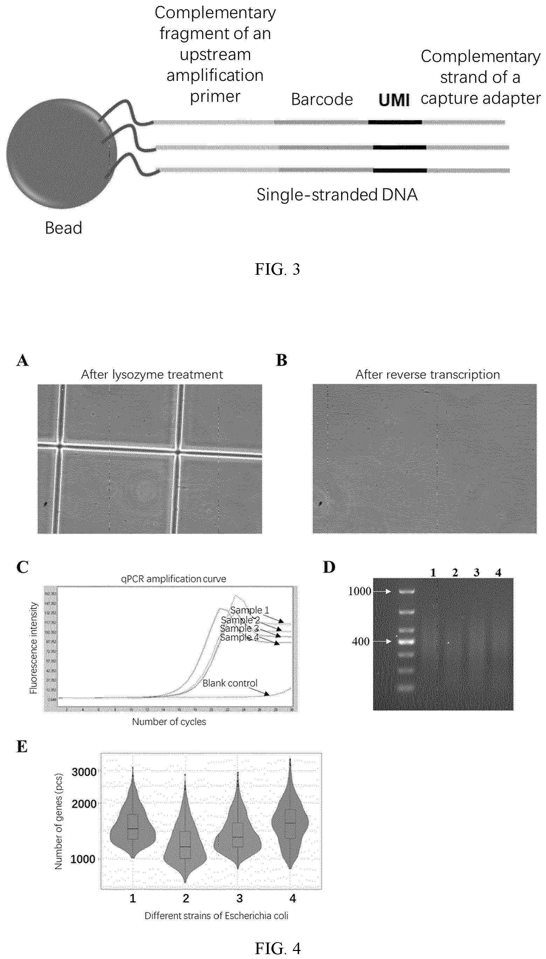

FIG. 3 is a schematic structural diagram of a coding bead used in the present disclosure.
FIG. 4 shows results of treating and sequencing of Escherichia coli samples in the method in the present disclosure. A. Escherichia coli under microscope whose cell wall is degraded by lysozyme; B. Escherichia coli under microscope that has been subjected to a reverse transcription reaction; C. Amplification curves of qPCR experiments; D. Diagram of nucleic acid gel electrophoresis; and E. Number of genes detected in Escherichia coli.

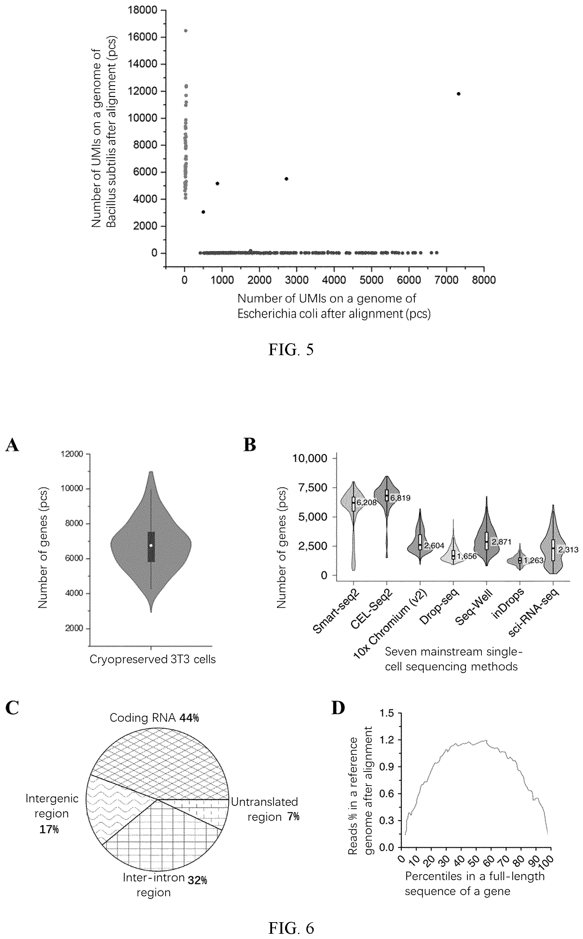

FIG. 5 shows results of sequencing a mixed sample of Escherichia coli and Bacillus subtilis in the method in the present disclosure; and the figure is a scatter diagram of numbers of UMIs on different strain genomes in a single cell after alignment, where the numbers of UMIs are numbers of cDNA molecules counted by sequencing, each dot in the figure denotes a cell, a cell denoted as a light dot contains almost only cDNA of Bacillus subtilis, a cell denoted as a dark dot contains almost only cDNA of Escherichia coli, and a cell denoted as a black dot is a contaminated cell.
FIG. 6 shows results of sequencing a mixed sample of human and mouse cell lines in the method in the present disclosure. A. Numbers of mouse single-cell genes detected by sequencing the mixed sample of human and mouse cell lines in the method in the present disclosure; B. Numbers of mouse single-cell genes detected by sequencing the mixed sample of human and mouse cell lines in seven existing sequencing methods, including the method of 10X Genomics; C. Distribution of sequencing reads of the mouse single-cell genes in sequencing results in different regions of a reference genome; and D. Statistical diagram of distribution uniformity of sequencing reads of the mouse single-cell genes in the sequencing results on a reference gene 5'-3'.

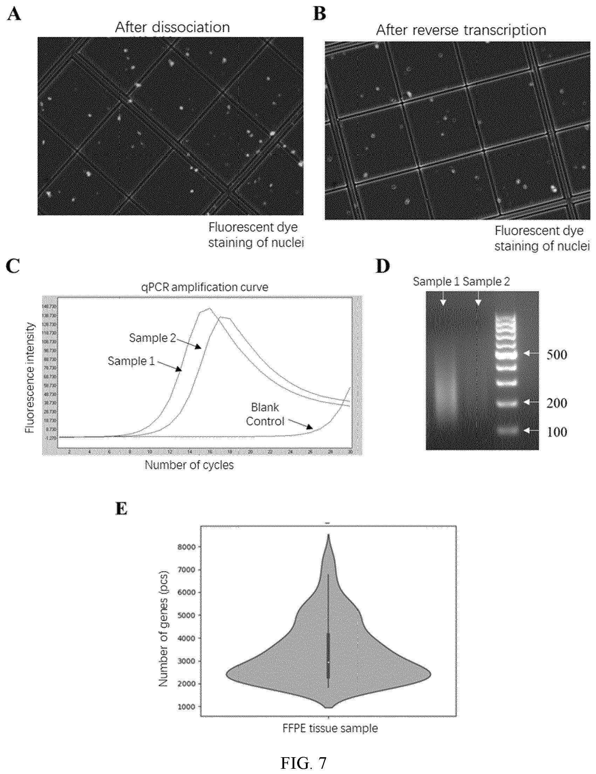

FIG. 7 shows results of sequencing a paraffin-embedded tissue sample of a mouse liver tissue in the method in the present disclosure. A. Nuclei of a dissociated mouse liver tissue under microscope; B. Nuclei of a mouse liver tissue under microscope that has been subjected to a reverse transcription reaction; C. Amplification curves of qPCR experiments; D. Diagram of nucleic acid gel electrophoresis; and E. Number of genes detected in the nuclei of the mouse liver tissue.

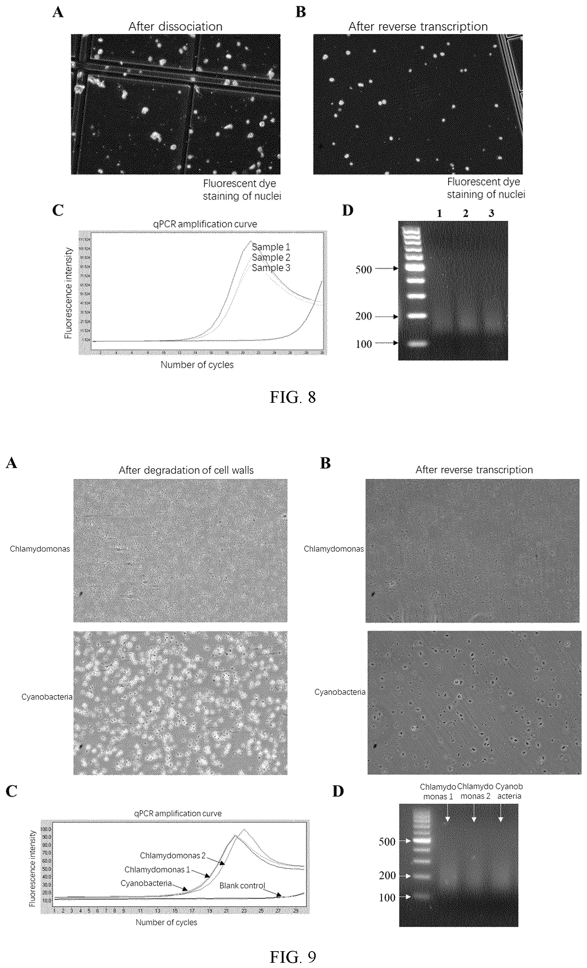

FIG. 8 shows results of making sequencing preparation for tobacco nucleus samples in the method in the present disclosure. A. Dissociated plant nuclei under microscope; B. Plant nuclei under microscope that have been subjected to a reverse transcription reaction; C. Amplification curves of qPCR experiments; and D. Diagram of nucleic acid gel electrophoresis.
FIG. 9 shows results of making sequencing preparation for Chlamydomonas and cyanobacteria samples in the method in the present disclosure. A. Chlamydomonas and cyanobacteria under microscope whose cell walls are degraded; B. Chlamydomonas and cyanobacteria under microscope that have been subjected to a reverse transcription reaction; C. Amplification curves of qPCR experiments; and D. Diagram of nucleic acid gel electrophoresis.

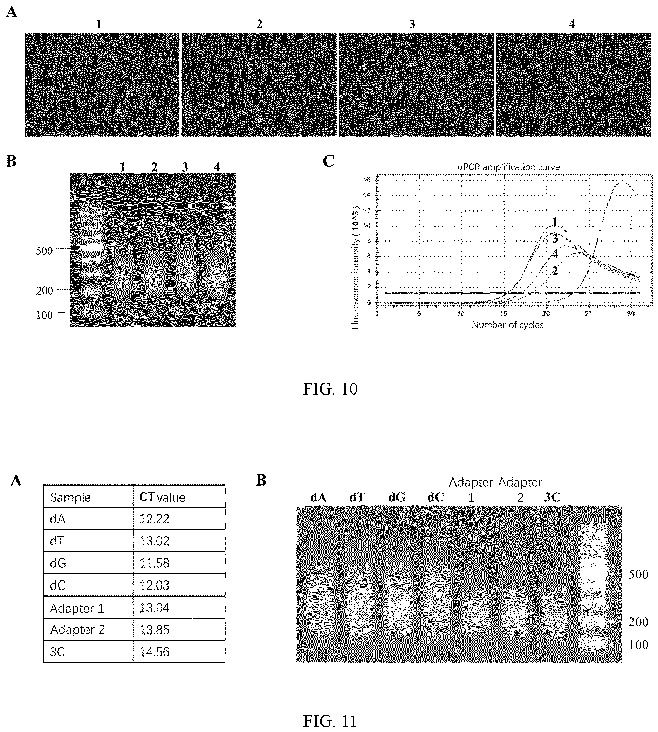

FIG. 10 shows results of making sequencing preparation for cell samples fixed with different types of fixative solutions in the method in the present disclosure. A. 3T3 cells under microscope that have been subjected to a reverse transcription reaction; B. Amplification curves of qPCR experiments; and C. Diagram of nucleic acid gel electrophoresis.
FIG. 11 shows results of making sequencing preparation for cell samples with different capture adapter fragments added to an end of cDNA strand in the method in the present disclosure. A. CT values of qPCR experiments; and B. Diagram of nucleic acid gel electrophoresis.

### Detailed description of the disclosure

Embodiments of the present invention provide a single-cell whole-RNA sequencing method. In the method, a reverse transcription primer is used to bind target RNA on a fixed single cell in an in situ reverse transcription reaction, then a capture adapter is added to the end of the first cDNA strand synthesized via reverse transcription, then a single chamber containing a single coding bead, a single cell and a reaction reagent is produced, the coding bead in the single chamber binds to the first cDNA via a complementary strand of the capture adapter and performs an elongation reaction, to synthesize the second cDNA strand with a barcode label, and finally, the obtained double-stranded cDNA is subjected to PCR amplification and high-throughput sequencing (as shown in FIG. 2). The sequencing method in this application can be combined with a microfluidic technique to establish a single-cell transcriptome sequencing platform with high throughput and high sensitivity.

### Definitions

Single cells in this specification include, but are not limited to, single cells or single nuclei of eukaryotic cells, prokaryotes (bacteria, actinomycetes, rickettsiae, chlamydiae, mycoplasma, cyanobacteria, archaebacteria, and the like), single-celled algae, viruses, and the like.

Cell samples in this specification include, but are not limited to, a fixed sample, a cryopreserved sample, a paraffin-embedded sample, and the like of cells.

RNA in this specification includes detectable coding RNA and various forms of non-coding RNA, for example, miRNA, IncRNA, siRNA and circRNA.

The barcode fragment in this specification refers to a string of base sequences used to distinguish between different cells, and as a cell tag, this base sequence needs to be stable, synthesizable, and highly variable. Generally, a suitable barcode fragment can be designed independently or selected from a barcode library. Barcode libraries of two mainstream single-cell sequencing techniques of 10X Genomics and Singleron Biotechnologies on the market can be found in their respective open-source quantitation software, Cellranger and CeleScope, respectively.

The random reverse transcription primer in this specification is formed by binding a complementary fragment of a downstream PCR amplification primer to a random 6-base sequence (that is, 5'-amplification primer fragment of complementary fragment of downstream amplification primer-NNNNNN-3', N = dG, dA, dT, or dC), where the random 6-base sequence can bind to the target RNA sequence, and the complementary fragment of the downstream amplification primer is used to bind to the downstream amplification primer in the PCR amplification reaction.

Main steps in the sequencing method in this application are summarized below.

### Preparation of coding beads

Single-stranded DNA on the coding beads in the present disclosure is formed by a complementary fragment of an upstream amplification primer, a barcode, a unique multiplex index (UMI), and a complementary strand of a capture adapter (referring to FIG. 3). The complementary fragment of the upstream amplification primer binds to the upstream amplification primer in the PCR amplification reaction. The barcode is used to label cDNA in the same cell. The UMI is a random sequence for labeling each original cDNA. The complementary strand of the capture adapter binds to the capture adapter attached to the first cDNA strand in a synthesis reaction of the second cDNA strand. As for the coding beads in this embodiment of the present invention, beads in single-cell sequencing kits of 10X Genomics, 1CellBio, Singleron Biotechnologies, BD Rhapsody, and the like may be used.

### Sample pretreatment

Preparation of single-cell suspensions: In this embodiment of the present invention, corresponding digestive enzymes can be selected correspondingly based on different types of cell samples to prepare the cell samples into the single-cell suspensions. For example, cultured cells can be digested into single cells by trypsin or EDTA. A fresh tissue can be digested by a corresponding digestive enzyme (for example, collagenase I and dispase are selected for a muscle tissue, and collagenase IV is selected for a liver tissue), and then filtered and washed to prepare into single cells. A cryopreserved sample first needs to be quickly thawed in a water bath at 25°C to 60°C. A paraffin-embedded sample first needs to be deparaffinized with xylene or another environmentally friendly dewaxing agent before being decrosslinked. For single-nucleus transcriptome sequencing, the single-cell sample first needs to be treated with a strong nonionic surfactant (NP-40, and the like), to lyse a cell membrane.

Fixation of single cells: In this embodiment of the present invention, a test cell sample is prepared into a single-cell suspension and then fixed with a fixative solution. Generally, when the sample is treated with the fixative solution, structures of macromolecules (RNA, proteins, and the like) inside the cells or nuclei are fixed, so that intact shapes, structures and composition of the single cells or nuclei can be maintained and RNA can be stably fixed in the cells or nuclei in subsequent experiments. During operation, appropriate fixative solutions can be selected according to characteristics of different types of samples. The fixative solutions include, but are not limited to, simple fixative solutions such as paraformaldehyde, formaldehyde, formalin, methanol, acetone, ethanol, acetic acid, picric acid, chromic acid, potassium dichromate and mercury bichloride, and the mixed fixative solution such as acetic acid-alcohol mixture, formalin-acetic acid-alcohol solution, and Bouin's fixative solution. Different fixing duration such as 15 minutes to 30 minutes or overnight is selected.

### Reverse transcription

In this embodiment of the present invention, the reverse transcription primers are bound to RNA of the fixed cells multiple times during in situ reverse transcription reactions, where the reverse transcription primers can be designed according to different actual requirements, and may be, for example, random reverse transcription primers or a combination of the random reverse transcription primer and Poly(dT) primer, or can also be target primers for specific genes or the like. 10% TritonX-10 can also be added into a reverse transcription system to permeabilize cell membranes of bacteria, so that a reaction reagent can more easily enter the bacteria.

### Addition of capture adapter

A capture adapter needs to be added to 3' end of the first cDNA strand after reverse transcription, so that the capture adapter can bind to a complementary strand of the capture adapter on the single-stranded cDNA of the coding beads, to further synthesize the second cDNA strand. In this embodiment of the present invention, Poly(dA), Poly(dT), Poly(dG), or Poly(dC) can be added as a capture adapter to the end of the first cDNA strand in a terminal transfer method; a specific capture adapter can also be added to the end of the first cDNA strand in a DNA ligation method; and three pieces of dC can also be added, as a capture adapter via a reverse transcriptase in a reverse transcription step in a template substitution method, to the end of the first cDNA strand obtained after reverse transcription. In contrast, the complementary strand of the capture adapter on the single-stranded cDNA of the coding beads can be adjusted correspondingly.

### Separation of single cells

In this specification, the separation of single cells generally means forming a single chamber that includes a single coding bead, a single cell, and a reaction reagent. To complete a subsequent elongation reaction of synthesizing the second cDNA strand, elongation reaction reagents in the single chamber usually include DNA polymerase, dNTPs, and a reaction buffer. In the method in the present disclosure, different microfluidic chips can be designed based on sizes and types of different cells, to generate microdroplets for the separation of single cells, or a microwell technique is used for the separation of single cells. Generally, based on the number of to-be-detected cells required for sequencing, the corresponding number of single droplets are collected or the corresponding number of microwells are prepared.

### Synthesis of second cDNA strand

The complementary strand of the capture adapter on the single-stranded cDNA attached to the coding bead binds to the capture adapter added to the first cDNA strand, and subsequently elongates to synthesize the second cDNA strand under action of DNA polymerase. The second strand of the obtained cDNA includes the complementary fragment of the upstream amplification primer, the barcode, the UMI, the complementary strand of the capture adapter, and the cDNA sequence.

### Library construction and high-throughput sequencing

Firstly, the original double-stranded cDNA obtained after the previous elongation reaction is purified in a magnetic bead method, and an upstream primer, a downstream primer and a reagent used for the PCR amplification reaction are added, to amplify the original double-stranded cDNA via PCR. A PCR amplification product is purified in the magnetic bead method, and the PCR amplification product is subjected to end repair, A-tailing, and adapter ligation in a TA-cloned adapter ligation-based library construction method. A constructed library can be subjected to high-throughput sequencing by using Illumina sequencing platform or BGI sequencing platform. The upstream primer and the downstream primer in the PCR amplification reaction are designed and synthesized based on the complementary fragment of the upstream amplification primer on the single-stranded cDNA of the coding bead and the complementary fragment of the downstream amplification primer in the random reverse transcription primer respectively.

### DETAILED DESCRIPTION

To make the objectives, technical solutions, and advantages of the examples of the present disclosure clearer, the following clearly and completely describes the technical solutions in the examples of the present disclosure with reference to the accompanying drawings in the examples of the present disclosure. Apparently, the described examples are some but not all of the examples of the present disclosure.

All other examples obtained by a person of ordinary skill in the art based on the examples of the present disclosure without creative efforts shall fall within the protection scope of the present invention.

Technical solutions of the present disclosure are further described in the following examples with reference to the accompanying drawings.

### Example 1: Sequencing of Escherichia coli samples

### Sample pretreatment

About 1 million Escherichia coli cells of each of different strains (1, 2, 3, and 4) were taken to verify that the solution of the present disclosure can be used for single-cell transcriptome sequencing of bacteria samples, the bacteria samples were washed with PBS buffer (PBST) containing 0.05% Tween-20 (Sangon Biotech (Shanghai) Co., Ltd.) three times, agglomerated bacteria were eliminated via shaking and filtration, so that bacteria in the sample formed a single-bacterium suspension. 1% paraformaldehyde (Beijing Solarbio Science & Technology Co.,Ltd.) was added, and a resulting mixture was fixed at 4°C overnight. After fixation, the bacteria samples were washed with PBST buffer three times, the cell walls were lysed via lysozyme (Thermo Fisher Scientific, United States), and the bacteria samples were treated at 37°C for 15 minutes, and washed with PBST buffer three times.

### Reverse transcription

Reverse transcription-permeabilization reaction reagents containing reverse transcriptase (Thermo Fisher Scientific, United States), a reverse transcription reaction buffer, dNTPs (Beijing Solarbio Science & Technology Co.,Ltd.), a random reverse transcription primer (Sangon Biotech (Shanghai) Co., Ltd.), and 10% TritonX-10 (Sangon Biotech (Shanghai) Co., Ltd.) were added into pretreated samples for the reverse transcription reaction, and each 50 µL of the reverse transcription reaction system was used for half a million single bacteria. After the reaction, the samples were washed with PBST buffer three times.

### Addition of capture adapter

Terminal transferase (Thermo Fisher Scientific, United States), dCTP (Sangon Biotech (Shanghai) Co., Ltd.), and a reaction buffer were added to the samples after reverse transcription, and a resulting mixture was incubated at 37°C for 30 minutes, so that a Poly(dC) capture adapter fragment was added to the 3' end of the first cDNA strand. After the reaction, the samples were washed with PBST buffer three times. The samples were subjected to microscopic examination, and the results showed that Escherichia coli samples could still remain in dispersed and intact single-bacterium morphology (referring to FIG. 4A) after complete lysis of cell walls and the reverse transcription reaction.

### Separation of single cells

Bacteria in each sample were counted and a bacteria concentration was adjusted to 200 pcs/µL, to 400 pcs/µL. Bacteria samples, elongation reaction reagents (including DNA polymerases (Thermo Fisher Scientific, United States), dNTPs, and a reaction buffer), coding beads (1CellBio, United States), and an oil phase (electronic fluorinated fluid 7500 containing 0.2% surfactant, 3M, United States) were added into a syringe, the syringe was connected with a corresponding inlet of the microfluidic chip through a hose, an appropriate flow rate was set to form a single water-in-oil droplet containing a single bacterium, a single coding bead and the elongation reaction reagent. About 200 µL of single droplets were collected.

### Synthesis of second cDNA strand

The collected single droplets were divided into different tubes for elongation reaction, to synthesize a second cDNA strand with a barcode label in a single droplet. After the elongation reaction ended, 20% PFO (containing 20% 1H, 1H, 2H, 2H-Perfluorooctanol electronic fluorinated fluid 7500, Sigma-Aldrich, United States) was added into each tube to lyse the single droplets, and the cDNA in the extraction tube was purified in the magnetic bead method (the beads were purchased from Beckman Coulter, United States).

### Library construction and high-throughput sequencing

Some double-stranded cDNA (200 to 400 cells) was used as a template in the qPCR experiment to detect total content of captured cDNA, and the results showed that an obtained CT value was about 17 (referring to FIG. 4B). The appropriate number of amplification cycles (generally equal to the CT value plus 3) was calculated based on the obtained CT value, and for cDNA samples in other remaining tubes, the coding cDNA was further amplified until suitable total content (about 100 ng) was achieved via the PCR amplification reaction. 3 µL of amplification product was subjected to nucleic acid electrophoresis, and the results showed that diffuse bands were obtained and sizes of the bands ranged mainly from 200 bp to 500 bp (referring to FIG. 4C), indicating that a high cDNA capture rate and wide coverage had been achieved for the Escherichia coli samples in the method in the present disclosure and the method can be used for subsequent high-throughput sequencing.

100 ng of amplified cDNA was subjected to end repair, A-tailing, and adapter ligation in the TA-cloned adapter ligation-based library construction method (library construction kit was purchased from Illumina, United States). A constructed library was subjected to high-throughput sequencing by using Illumina sequencing platform. The sequencing results showed that the numbers of detectable genes in a single Escherichia coli cell ranged from 1000 to 3000 (referring to FIG. 4E), that is, basically all the genes expressed in Escherichia coli were contained, indicating that the method in the present disclosure can be used for single-cell transcriptome sequencing of bacteria while ensuring high sensitivity.

### Example 2: Sequencing of mixed sample of Escherichia coli and Bacillus subtilis

A mixed sample of about 1 million Escherichia coli and Bacillus subtilis cells was taken to verify accuracy of the solution of the present disclosure in single-cell transcriptome sequencing. The method in the foregoing Example 1 was used for sample pretreatment, reverse transcription, capture adapter, isolation of single cells, synthesis of the second cDNA strand, library construction, and high-throughput sequencing. Sequencing results showed that a total of 251 bacteria were detected in the mixed samples of Escherichia coli and Bacillus subtilis after genome alignment, and 4 bacteria contained cDNA of both Escherichia coli and Bacillus subtilis, which was usually caused because two bacteria entered the same droplet; and the other bacteria almost exclusively contained either cDNA of Escherichia coli or cDNA of Bacillus subtilis, and an overall contamination rate was probably less than 2% (referring to FIG. 5). It was indicated that the solution of the present disclosure was of high accuracy in the single-cell transcriptome sequencing, and could ensure that the mixed Escherichia coli and Bacillus subtilis in the sample could be well isolated and few cross-contamination between species occurred.

### Example 3: Sequencing of mixed samples of human and mouse cell lines

A mixed sample of about 1 million cells of human and mouse cell lines (50% human HEK293 cells and 50% mouse 3T3 cells) cryopreserved at -80°C were taken and quickly thawed in a 37°C water bath, and washed with PBS buffer three times, 1% paraformaldehyde was added, and a resulting mixture was fixed overnight at 4°C. After fixation, cryopreserved cell samples were washed with PBST buffer three times. Then, with reference to the method in the foregoing Example 1, capture adapter, isolation of single cells, synthesis of the second cDNA strand, library construction, and high-throughput sequencing were performed. Data of mouse 3T3 cells was separated after genome alignment, and the results showed that in the method in the present disclosure, the numbers of detectable mouse single-cell genes in cryopreserved cell samples ranged from 4000 to 10000 (referring to FIG. 6A). As reported in Systematic Comparison of Single-Cell and Single-Nucleus RNA-Sequencing Methods (Jiarui Ding et al., Nat Biotechnol. 2020 June; 38(6): 737-746.), the mixed samples of human and mouse cell lines (50% human HEK293 cells and 50% mouse 3T3 cells) were sequenced in seven single-cell transcriptome sequencing methods (including 2 microwell-based low-throughput methods (Smart-seq2 and CEL-Seq2) and 5 high-throughput methods (10x Chromium, Drop-seq, Seq-Well, inDrops, and sci-RNA-seq)) in prior art. After comparison between mouse single-cell sequencing results detected in the method in the present disclosure and mouse single-cell sequencing data detected in the foregoing seven methods, under the same sequencing depth, sensitivity of the sequencing method in the present disclosure was equivalent to that of the low-throughput single-cell sequencing methods such as Smart-seq2, and was much greater than that of high-throughput single-cell sequencing methods such as 10X Genomics (10X Chromium) (referring to FIG. 6B). Sequencing alignment results showed that sequencing reads of mouse single cells were distributed in different regions of the reference genome, including coding RNA, an intergenic region, an inter-intron region, and an untranslated region (referring to FIG. 6C), indicating that the whole transcriptome can be sequenced via the solution of the present disclosure. The sequencing alignment results showed that the sequencing reads of the mouse single cells were evenly distributed from 5' end to 3' end of the reference gene (referring to FIG. 6D).

### Example 4: Sequencing preparation of paraffin-embedded tissue sample of mouse liver tissue

About 20 mg of the paraffin-embedded samples of the mouse liver tissue were taken and first deparaffinized and rehydrated, a cell lysate solution was added to lyse the tissue into single nuclei, and a resulting mixture was washed with PBST three times. Then, with reference to the method in the foregoing Example 1, capture adapter, isolation of single cells, synthesis of the second cDNA strand, library construction, and high-throughput sequencing were performed. The results showed that single dispersed nuclei successfully isolated in the FFPE samples could still remain in scattered and intact single-nucleus morphology after the reverse transcription reaction (referring to FIG. 7A and FIG. 7B). After the purified double-stranded cDNA product was obtained, some cDNA (about 100 nuclei) was used as a template to conduct the qPCR experiment, total content of captured cDNA was detected, and an obtained CT value was about 10 (referring to FIG. 7C), indicating that a high cDNA capture rate had been achieved for single nuclei in the FFPE samples in the method in the present disclosure. The cDNA product was subjected to PCR amplification (15 cycles) based on the CT value. 3 µL of amplification product was subjected to nucleic acid electrophoresis, and diffuse bands were obtained and sizes of the bands ranged mainly from 200 bp to 500 bp (referring to FIG. 7D), indicating that a high cDNA capture rate and wide coverage had been achieved for the single nuclei in the FFPE samples in the method in the present disclosure and the method can be used for subsequent sequencing. Sequencing results showed that 2000 to 3000 genes in a single nucleus in the FFPE samples can be detected in the method in the present disclosure (referring to FIG. 7E), indicating that the method in the present disclosure can be used for single-nucleus transcriptome sequencing of the FFPE samples.

### Example 5: Sequencing preparation for tobacco nucleus samples

Fresh tobacco samples were dissociated into single cell nuclei by a single-cell preparation instrument (referring to FIG. 8A), about 1 million tobacco nucleus samples were taken and washed with PBS buffer three times, 1% paraformaldehyde was added, and a resulting mixture was fixed at 4°C overnight. After fixation, tobacco nucleus samples were washed with PBST buffer three times. Then, with reference to the method in the foregoing Example 1, reverse transcription, addition of the capture adapter, isolation of single cells, synthesis of the second cDNA strand, and library construction were performed. The results showed that tobacco nucleus samples could still remain in scattered and intact single-nucleus morphology after the reverse transcription reaction (referring to FIG. 8B). A CT value of the tobacco nuclei obtained via the qPCR experiment was about 16 (referring to FIG. 8C), indicating that more cDNA of the tobacco nucleus samples can be captured in the method in the present disclosure. As a result of nucleic acid gel electrophoresis, diffuse bands were obtained and sizes of the bands ranged mainly from 150 bp to 300 bp (referring to FIG. 8D), indicating that a high cDNA capture rate and wide coverage had been achieved for single cells in the tobacco nucleus samples in the method in the present disclosure and the method can be used for subsequent sequencing.

### Example 6: Sequencing preparation for mixed samples of Chlamydomonas and cyanobacteria

About 1 million Chlamydomonas samples and 1 million cyanobacteria samples were taken separately to verify that the solution of the present disclosure can be used for single-cell transcriptome sequencing of Chlamydomonas and cyanobacteria samples. The Chlamydomonas and cyanobacteria samples were washed with PBS buffer three times, 1% paraformaldehyde was added, and a resulting mixture was fixed at 4°C overnight. After fixation, the Chlamydomonas and cyanobacteria samples were washed with PBST buffer three times. A cell wall enzymolysis solution (pH = 6.5) containing 4% cellulase, 0.5% pectinase, and 0.3 mol/L mannitol was added into the Chlamydomonas samples, and a resulting mixture was treated at 37°C for 15 minutes, and washed with PBST buffer three times. Lysozyme was added into the cyanobacteria samples to lyse cell walls, and a resulting mixture was treated at 37°C for 15 minutes, and washed with PBST buffer three times. Then, with reference to the method in the foregoing Example 1, the Chlamydomonas and cyanobacteria samples whose cell walls were lysed were subjected to reverse transcription, addition of the capture adapter, isolation of single cells, synthesis of the second cDNA strand, and library construction. The results showed that the Chlamydomonas and cyanobacteria samples could still remain in dispersed and intact single-cell morphology (referring to FIG. 9A and FIG. 9B) after complete lysis of cell walls and the reverse transcription reaction. A CT value of the Chlamydomonas samples obtained via the qPCR experiment was about 17, and a CT value of the cyanobacteria samples was about 17 (referring to FIG. 9C), indicating that high cDNA capture rates had been achieved for both the Chlamydomonas and cyanobacteria samples in the method in the present disclosure. As a result of nucleic acid gel electrophoresis, diffuse bands were obtained and sizes of the bands ranged mainly from 200 bp to 500 bp (referring to FIG. 9D), indicating that a high cDNA capture rate and wide coverage had been achieved for single cells in the Chlamydomonas and cyanobacteria samples in the method in the present disclosure and the method can be used for subsequent sequencing.

### Example 7: Sequencing preparation for mouse 3T3 cell line samples (using different fixative solutions and different capture adapters)

Four groups of cultured mouse 3T3 cell lines were separately taken to verify treatment effects of different fixative solutions on samples. Trypsin was added into a cell culture dish for digestion at 37°C for 3 minutes to digest the samples into single cells. A resulting mixture was washed with PBS buffer three times, 1% paraformaldehyde, 70% ethanol, acetone, and 1% acetic acid (samples 1, 2, 3, and 4) were added separately, a resulting mixture was fixed at 4°C overnight, and after fixation, the resulting mixture was washed with PBST three times. Then, with reference to the method in the foregoing Example 1, reverse transcription, addition of the capture adapter, isolation of single cells, synthesis of the second cDNA strand, and library construction were performed. The results showed that cell samples fixed with different types of fixative solutions could can all remain in intact single-cell morphology after the reverse transcription reaction (referring to FIG. 10A). A high cDNA capture rate had been achieved for cell samples fixed with different types of fixative solutions in the method in the present disclosure (referring to FIG. 10B). Diffuse bands were obtained and sizes of the bands ranged mainly from 200 bp to 500 bp (referring to FIG. 10C), indicating that a high cDNA capture rate and wide coverage had been achieved for the cell samples fixed with different types of fixative solutions in the method in the present disclosure and all the fixative solutions can be used for subsequent high-throughput sequencing.

In addition, 7 groups of cultured mouse 3T3 cell lines were separately used to verify an effect of adding different capture adapters to the end of the first cDNA strand. Trypsin was added into a cell culture dish for digestion at 37°C for 3 minutes to digest the samples into single cells. A resulting mixture was washed with PBS buffer three times, 1% paraformaldehyde was added, a resulting mixture was fixed at 4°C overnight, and after fixation, the resulting mixture was washed with PBST three times. 6 groups of fixed cell samples were taken and subjected to the reverse transcription reaction in the method in the foregoing Example 2. The dATPs, dTTPs, dGTPs, and dCTPs, as well as the terminal transferase and the reaction buffer, were added into the 4 groups of cell samples after the reverse transcription ended, a resulting mixture was incubated at 37°C for 30 minutes, and Poly(dA), Poly(dT), Poly(dG), and Poly(dC) were added to the end of cDNA as capture adapter fragments, respectively. DNA ligase, specific capture adapter fragments, and the reaction buffer were added into 2 other groups of cell samples after the reverse transcription ended, a resulting mixture was incubated at 37°C for 30 minutes, and specific capture adapter fragments were added to the end of cDNA. The reverse transcription-permeabilization reaction reagent containing reverse transcriptase (Moloney Murine Leukemia Virus), the random reverse transcription primer, dNTPs, the reverse transcription reaction buffer, and 10% TritonX-10 was added to another group of cell samples after fixation ended, and three pieces of dC were added to the end of the cDNA as capture adapter fragments after the reverse transcription ended. With reference to the method in the foregoing Examples 4 to 6, cell samples with capture adapters added were subjected to isolation of single cells, synthesis of the second cDNA strand, and library construction. The results showed that CT values of cell samples with different capture adapter fragments were between 12 and 13, indicating that more cDNA in the cells can be captured in the method in the present disclosure by using different capture adapter fragments (referring to FIG. 11A). As a result of nucleic acid gel electrophoresis, diffuse bands were obtained and sizes of the bands ranged mainly from 200 bp to 500 bp (referring to FIG. 11B), indicating that a high cDNA capture rate and wide coverage had been achieved for cells in the method in the present disclosure by using different capture adapter fragments and all the capture adapter fragments can be used for subsequent high-throughput sequencing.

The basic principles, main features, and advantages of the present disclosure are shown and described above. A person skilled in the art should understand that the present invention is not limited to the foregoing examples, and the descriptions in the foregoing examples and specification only illustrate the principles of the present invention. Various changes and improvements can also be made to the present invention without departing from the spirit and scope of the present invention, and these changes and improvements shall fall within the claimed scope of the present invention. The claimed protection scope of the present invention is subject to the appended claims and equivalents thereof.

## Claims

1. A single-cell transcriptome sequencing method comprising:
preparing a single-cell suspension with a test cell sample and then fixing cells with a fixative solution; and
synthesizing a first cDNA strand using by performing an in-situ reverse transcription reaction on RNA of the fixed single cell using a reverse transcription primer.

2. The method of claim 1, wherein the reverse transcription primer is a random reverse transcription primer, a reverse transcription primer designed for a target RNA sequence, or a mixture of the random reverse transcription primer and the reverse transcription primer designed for the target RNA sequence.

3. The method of claim 1 or 2, further comprising adding a capture adapter to an end of the first cDNA strand obtained by the reverse transcription, wherein the capture adapter is sequence-complementary to a complementary strand of the capture adapter on a coding bead, and the capture adapter is a random fragment of a known sequence, preferably a Poly(dA) fragment, a Poly(dT) fragment, a Poly(dG) fragment, or a Poly(dC) fragment.

4. The method of claim 3, further comprising embedding the single cell and the single coding bead into a single chamber after adding the capture adapter, to separate the single cell and synthesize a second cDNA strand in the single chamber.

5. The method of claim 4, further comprising amplifying double-stranded cDNA via PCR, constructing a library and performing sequencing, after the second cDNA strand is synthesized.

6. The method of any one of claims 3 to 5, wherein a single-stranded DNA on the coding bead used in the method comprises a complementary fragment of an upstream amplification primer, a barcode, a UMI, and a complementary strand of the capture adapter; and preferably, the barcode comprises one or more barcodes, or preferably, three barcodes.

7. The method of any one of claims 3 to 6, wherein the separation of the single cells is completed by a microfluidic chip or a microwell used in the method.

8. The method of any one of claims 1 to 7, wherein the fixative solution is a simple fixative solution or a mixed fixative solution; preferably, the simple fixative solution comprises, but is not limited to, paraformaldehyde, formaldehyde, formalin, methanol, acetone, ethanol, acetic acid, picric acid, chromic acid, potassium dichromate and mercury bichloride; and preferably, the mixed fixative solution comprises, but is not limited to, acetic acid-alcohol mixture, formalin-acetic acid-alcohol solution, and Bouin's fixative solution.

9. Use of the method of any one of claims 1 to 8 in whole-transcriptome sequencing of a single cell, a single nucleus, and a single microorganism; and preferably, the use is the use in fields of microbiology, basic medicine, clinical medicine, agronomy, cell biology, immunology, developmental biology, pathology, neurobiology and neurodevelopment, genetics, stem cells, tumors, reproductive health, metagenomics, microecology, and new drug development.
